# EUROPEAN PATENT APPLICATION

(11) **EP 4 491 269 A1**
(43) Date of publication of application: **15.01.2025**
(21) Application number: 23184917.5
(22) Date of filing: 12.07.2023
(51) Int. Cl.: B01J 21/12, B01J 23/755, B01J 37/00, B01J 37/02, B01J 37/03, B01J 37/08, C12P 7/6463

(54) **CATALYST FORMULATION FOR DIRECT CONVERSION OF MICROALGAE IN GROWTH MEDIUM TO BIOFUELS**

(71) Applicant: Socar Turkey Arastirma Gelistirme Ve Inovasyon A.S., Istanbul (TR)
(72) Inventor: Deliismail, Ozgun, Izmir (TR)
(74) Representative: Sevinç, Cenk

(57) **Abstract**

This invention relates to catalyst formulation to be used in direct conversion of *Nannochloropsis oculata* marine microalgae into biofuels and biochemicals without harvesting and dewatering and synthesis method of this formulation. In this method, nickel on silica/alumina (Ni-Al₂O₃-SiO₂) catalyst mixture formulation is obtained by using single step sol-gel method. The catalyst mixture formulation percentages to obtain high catalytic activity and selectivity are 70% Al₂O₃, 30% SiO₂ and 10% Ni by weight.

## Description

### Technical Field of the Invention

Present invention relates to a catalyst formulation to be used in direct conversion of *Nannochloropsis oculata* marine microalgae into biofuels and biochemicals without harvesting and dewatering and also synthesis method of the catalyst formulation. In this method, nickel on silica/alumina (Ni-Al₂O₃-SiO₂) catalyst formulation is obtained by using single step sol-gel method.

### State of the art

Biofuels which are produced from biomass such as vegetable oils, animal waste are gaseous or liquid renewable fuels, and generally used as transportation fuels [1], and they are regarded as one of the most viable options for reducing CO₂ emissions in the transport sector since it's more sustainable than its fossil fuel counterpart which has a finite resource. Use of biofuels has also beneficial advantage of significantly reducing particulate formations, which otherwise is released into the air, leading to better air quality and far less pollution. According to the report published by researchers from Harvard University, burning fossil fuels like coal and diesel was responsible for about 1 in 5 deaths worldwide [2]. In addition, biofuels offer high quality performance, and can be used in existing engines and provides them to last longer as they don't have a high rate of viscosity. When all the advantages of biofuels are taken into consideration, using biofuels in industrial purposes have many opportunities over current and general source of non-renewable fuels, and in order to experience these advantages, biofuels are generated from biomass like vegetable oils, agricultural residues, algae etc. However, use of these types of biomass have some limitations that procurement of oily plant seed can be rough, thus the production of biofuels using microalgae have many advantages, for instance, high production rate of microalgae per hectare per year, salty or waste water usage instead of using fresh water, easy cultivation, no requirement for agricultural land, high biomass yield, over conventional production methods using other varieties.

In the prior art, there are studies on conversion of microalgae into biofuels using different methods and/or catalyst combinations. Generally, these methods involve harvesting and dewatering of microalgae before using in a conversion process. The harvesting is an essential process that it should be effective for the majority of microalgal strains, and also should allow the achievement of high biomass concentrations [3], and microalgae in growth medium, which contains mainly water, is 3-10 wt.% and there is a necessity to increase this percentage to minimum 20 wt. % for the further biofuel production processes using wet microalgae, but the cost of the harvesting of microalgae biomass from growth medium accounts for 20-30% of the total production cost due to the low biomass production and the small size of cells when they are cultured in liquid medium. The dewatering process is also as costly as the harvesting since when dewatering procedure is applied, the drying of microalgal paste or cultures result in extremely high operational costs [4]. Beside these limitations related with harvesting and dewatering processes during currently used approaches, general methods for the conversion of microalgae into biofuels are thermochemical and biochemical processes. Pyrolysis is a thermochemical conversion technique that typically works with dry algae at 400-600 °C under air or inert gas flow. Meanwhile, sub- or supercritical water is necessary in the liquefaction method to convert 20 wt.% wet microalgae to bio-oil. Thus, the cost and energy intensive necessities make these thermal methods unviable for continuous large-scale biofuel production from microalgal biomass. On the other hand, biochemical methods such as anaerobic digestion and fermentation need long time to produce methane or ethanol since these methods are not feasible as well for continuous industrial processes. In the literature, there have been some alternative studies in which enzymatic and chemical pretreatments were applied on microalgae, to release the carbohydrates from algal cell. Harun, Danquah and Ho et al. reported the disruption of algal cell walls and hydrolysis carbohydrates to monosaccharides in acidic medium for which sulfuric acid was used but it is homogeneous process and this leads to another economic disadvantage factor in which the acidic reaction medium has to be washed to remove sulfuric acid and adjust pH.

In the prior art, a method was proposed by Cheng and et al. [5] for the direct conversion of wet microalgae biomass into biodiesel by microwave irradiation in one step, thus set back the need for harvesting of microalgae, and extreme high costs which comes with that. Biomass (water content 80% by weight) was placed in the digestion reactor and mixed with chloroform, methanol and sulfuric acid. The digestion reactor was then closed and heated to the set temperature by microwave radiation. The algal biomass was kept at the set temperature as a function of curing reaction time. However, the proposed one step method does not involve any use of solid catalyst, only sulfuric acid as a homogeneous catalyst, and the method is based on transesterification, so it'd be problematic to be removed after the reaction compare to the solid catalysts, also it requires a controlled washing which depends on pH control, resulted in high procedure cost and unnecessary transaction. In order to prepare catalysts, sol-gel techniques are known to be intriguing ways. The diversity of sol-gel processes makes it feasible to produce customized materials such disseminated metals, oxidic catalysts, and chemically altered supports while controlling the texture, content, homogeneity, and structural features of solids [5].

Recently, Zuorro and et al. [6] reported that one promising method for thermochemically converting biomass into more usable liquid fuels is hydrothermal liquefaction (HTL) carried out at high pressures and temperatures. Contrary to pyrolysis, HTL may produce biocrude from high-moisture biomass in a water media, eliminating the need for preparatory drying procedures. However, biocrude derived from algae has drawbacks such as high water content, high viscosity and high heteroatom content, making it difficult to upgrade to a usable fuel. The application of catalysts in HTL reactions is an interesting possibility to improve the process in several aspects such as the yield and quality of bio-crude, suppression of side reactions, reduction of reaction temperature and pressure, reduction of viscosity and processing time [6], thus heterogeneous catalysts which are used in the conversion of microalgae into biofuels during hydrothermal liquefaction process in the state of the art are more advantageous over homogeneous catalysts due to their low corrosion rate and high catalytic activity under severe reaction conditions, which frequently harm the homogeneous catalysts.

In the prior art, there are problems such as high operational cost during the harvesting and dewatering processes, high-energy requirements (centrifugation), contamination with addition of electrolyte/cationic polymers (coagulation, flocculation) or non-feasible scale-up procedures, low quality of biofuels derived from biomass. Because of described problems in prior art, developments are required to overcome these problems.

### Brief Description and Objects of the Invention

In the present invention, a catalyst formulation to be used in direct conversion of *Nannochloropsis oculata* marine microalgae into biofuels without harvesting and dewatering and synthesis method of this formulation is disclosed. In the synthesis method, nickel on silica/alumina (Ni-Al₂O₃-SiO₂) catalyst formulation is obtained by using single step sol-gel method. The catalysts that are the subject of the invention are solid heterogeneous catalysts and perform the synthesis of biofuels and biochemicals from algal biomass in growth medium which contains *Nannochloropsis oculata* species and thus, the synthesis of biofuel can be done much simpler and the operational cost is significantly reduced with the use of the solid catalyst since it eliminates the harvesting and dewatering of microalgae. In addition to this, seawater used in microalgae growth medium contains sodium chloride which has no adverse effects on the activity and selectivity performance of the catalyst.

The most important object of the present invention is to enable a sol-gel method to prepare heterogeneous catalyst formulation for use in biofuels/biochemicals production from wet microalgae. The sol-gel synthesis method of the invention differs the current sol-gel methods in such a way that the formation of mixed oxides' physicochemical properties, such as acidity and acidic strengths in addition to surface area, crystalline phases etc., could be tailored to obtain the highly active and selective catalyst without being affected by the presence of large amount of salt in seawater of the microalgae growth medium. Not only the ratios (or magnitude) of the parameters, such as H₂O/(Al or Si) or acid type/Si or temperatures, used in the sol-gel method of the invention, but also the order and duration of mixing of precursors used in the sol-gel of the invention significantly affects the activity and selectivity of the catalyst of the invention. In the present invention, sulfuric acid and its molar ratios given in the Example I are necessary to obtain stable alumina sol. Even though the ratios and magnitudes of the parameters, such as acid molar ratio, water molar ratio etc., are generally known, they cannot directly be used for any catalyst formulation. Most importantly, the order and duration of mixing of the precursors (especially sulfuric acid) of the sol-gel method of the invention have never been considered in a classical sol-gel method. In fact, the sol-gel synthesis method of the present invention is specifically designed for the catalyst formulation used in direct conversion of microalgae in seawater to biofuels and biochemicals without harvesting and dewater. Thus, the parameters are unique to the catalyst formation and also formation of stable alumina sol when sulfuric acid is used. Through optimized parameters used in the synthesis method of this formulation, synthesized catalyst is able to convert wet algae directly into biofuel and biochemicals without the requirement of harvesting and dewatering.

Another object of present invention is to provide a heterogeneous catalyst which eliminates requirement of complex processes, which are harvesting and dewatering, and extra costs in biofuel and biochemical production from wet microalgae. In other words, the feature of the alumina-silicate supported nickel catalyst is that it actually reduces operational costs which are the results of harvesting and dewatering processes, and complex process requirements due to the harvesting and dewatering. Mentioned heterogeneous catalyst can enable to acid-hydrolysis reaction to occur and break C-C bonds via water where microalgae are located, thus, while performing hydrolysis reaction, simultaneously microalgae's cell wall are ruptured in order to be appeared lipids which belong to microalgae itself. Therefore, with the use of object of the invention, catalyst, emerged lipids from microalgae can be converted to desired products with high selectivity. In the present invention, the direct conversion of wet microalgae to biofuels and biochemicals was as an example carried out in a batch reactor with addition of 0.25 g of a catalyst, Ni-Al₂O₃-SiO₂. Also, the conversion of microalgae to biofuels and biochemicals occurs in the growth media, which is maintained at low pressure (around 1 atm) and low temperature (below 150 degrees). Therefore, multiple handling, and consequently numerous steps are not necessary while conversion to biofuel proceeds.

Another object of the present invention is the production of biofuel and biochemicals with green technology. In fact, biofuel production in the present invention are made with the conversion of microalgae. Therefore, environmental negative impacts related to fuel usages can be disposed with the biofuels produced with the approach of the current invention.

Present invention overcomes the problems present in the prior art by preventing requirement of complex processes and extra operational costs in biofuel production from wet algae, offering a heterogeneous catalyst which shows activity at low pressure and low temperature. With the help of the catalyst of the invention, biofuel production can be achieved without harvesting and dewatering the microalgae. Thus, operational cost related to harvesting and dewatering of the microalgae is significantly reduced. Also, microalgae growth medium is made up of seawater but the catalyst's activity is not adversely affected by the presence of sodium chloride or any ingredients of the growth medium.

### Description of the Figures

**Figure 1****.** The crystalline phases and average crystallite sizes of the catalysts are determined by X-Ray Diffractometer
**Figure 2****.** Volcano curve for Nannochloropsis oculata conversion (%) as a function of acidity (µmol/g) (a) the catalyst prepared with sulfuric acid, (b) The catalyst prepared with sulfuric acid (after processed in seawater), (c) the catalyst prepared with HNO3, (d) the catalyst prepared with HCl
**Figure 3****.** The conversion of *Nannochloropsis oculata* (a) 10% Ni - 30% Al₂O₃-70% SiO₂- H₂SO₄-SW, (b) 10% Ni-30% Al₂O₃-70% SiO₂-H₂SO₄, (c) 10% Ni-30% Al₂O₃-70% SiO₂-HNO₃, (d) 10% Ni-30% Al₂O₃-70% SiO₂-HCl

### Detailed Description of the Invention

This invention relates to heterogeneous catalyst formulation and its preparation using the corresponding sol-gel method to be used in the direct conversion of *Nannochloropsis oculata* marine microalgae into biofuels without harvesting and dewatering and synthesis method of this formulation. In the synthesis method, nickel on silica/alumina (Ni-Al₂O₃-SiO₂) catalyst mixture is obtained by using single step sol-gel method. The catalysts that are the subject of the invention are heterogeneous and perform the production of biofuels and biochemicals from wet microalgae, and thus, the production of biofuels and biochemicals can be done easily and economically. In addition to this, seawater used in microalgae growth medium does not adversely affect the activity of the catalyst. The catalyst formulation weight percentages are 5-95% Al₂O₃, 95-5% SiO₂ and 0.5-30% Ni by weight (the best formulation to obtain high activity and selectivity is 70% Al₂O₃-30 %SiO₂-10 %Ni).

For direct conversion of microalgae into biofuels and biochemicals without harvesting and dewatering, the heterogeneous catalyst formulation synthesized using a one-step sol-gel method of the invention comprises the following steps:
i. synthesizing silica sol by mixing 1-5 M hydrochloric acid (HCl, 36.5-38 wt.%) with the precursor of tetraethylorthosilicate (≥ 98% purity), ethanol (≥99.8 % purity), and deionized water at 60-90 °C for 1-4 hours
ii. synthesizing alumina sol by mixing precursor of aluminum isopropoxide (AIP, ≥ 98% purity), deionized water at room temperature,
iii. increasing the temperature to 80-90°C in 15-30 min with 1000-1500 rpm of stirring rate and adding sulfuric acid (H₂SO₄, ≥98 purity) or hydrochloric acid or nitric acid (68 wt.%) to obtain the alumina sol,
iv. mixing silica and alumina sols, and adding nickel (II) acetate hydrate (>99% purity) to the silica-alumina sol mixture,
v. evoporating the excess solvent at 50-80°C under vacuum or inert flow for 30-120 min, and obtaining a gel,
vi. drying the gel at 100-150°C for 3-24 h, and calcinating the gel at 500-900°C for 6-24 h with 10°C/min heating rate, obtaining a catalyst which has formulation of Ni-Al₂O₃-SiO₂
vii. after calcination, ball milling or hand grinding and sieving of the catalyst to mesh size less than 200 for catalytic reactions
viii. sieved catalysts are pretreated with sodium chloride (1-5 wt.%) containing water at 70-90 °C for 12-48 h, and drying at 100-150 °C for 3-24 h.

One example of the embodiment of the one-step sol-gel method of the invention comprises the sol-gel parameters and the following sol-gel steps to obtain high catalytic activity and selectivity:

### Sol-gel parameters:

H₂O/Al (mol): 115.5
H₂SO₄/Al (mol): 2.42
EtOH/Si (mol):3.81
H₂O/Si (mol): 4.69
HCl/Si (mol): 7.30×10⁻⁴

Another embodiment of the invention, for direct conversion of microalgae into biofuels and biochemicals without harvesting and dewatering, the heterogeneous catalyst formulation synthesized using a one-step sol-gel method of the invention comprises the following steps:
i. synthesizing silica sol by mixing 1 M hydrochloric acid (HCl, 38 wt.%) with the precursor of tetraethylorthosilicate (≥ 98% purity), ethanol (≥99.8 % purity), and deionized water at 80 °C for 2 hours
ii. synthesizing alumina sol by mixing precursor of aluminum isopropoxide (AIP, ≥ 98% purity), deionized water at room temperature,
iii. increasing the temperature to 85°C in 30 min with 1500 rpm of stirring rate and then adding sulfuric acid (H₂SO₄, ≥98 purity) or hydrochloric acid or nitric acid (68 wt.%),
iv. mixing silica and alumina sols, and adding nickel (II) acetate hydrate (>99% purity) to the sol mixture,
v. evoporating the excess solvent at 70°C, and obtaining a gel,
vi. drying the gel at 120°C for 12 h, and calcinating the gel at 900°C for 6 h with 10°C/min heating rate, obtaining a catalyst which has formulation of Ni-Al₂O₃-SiO₂,
vii. after calcination, grinding and sieving of the catalyst to mesh size of less than 200 mesh for catalytic reactions,
viii. sieved catalysts are pretreated with sodium chloride (3.5 wt.%) containing water at 90 °C for 24 h, and drying at 120 °C for 12 h.

H₂O/Al proportion is used to calculate the amount of water and aluminum isopropoxide in step ii, H₂SO₄/Al proportion is used to calculate the amount of sulfuric acid and alumina in step iii, EtOH/Si proportion is used to calculate the amount of ethanol and silica in step i, H₂O/Si proportion is used to calculate the amount of water and silica in step i, and HCl/Si proportion is used to calculate the amount of hydrochloric acid and silica in step i.

Si sol and Al sol are synthesized separately, as can be seen in steps i and ii since the hydrolysis reaction rates of the two starting materials, TetraEthyl OrthoSilicate (TEOS) and aluminum isopropoxide, are significantly different, thus having distinct requirements for sol-gel procedure. As a result of completing the hydrolysis in separate environments, one of the Al₂O₃ or SiO₂ phases is prevented from dominating during gelation after mixing. The formation of a homogeneous Al₂O₃ and SiO₂ mixture are ensured by preparing sol in separate environments with the described parameters above before mixing.

As can be seen from step i, to be able to synthesize the Silica sol, using tetraethyl orthosilicate (TEOS), temperature, and mixing time, as well as the added amounts of ethanol, HCl, and water, have all been calculated explicitly for the intended catalyst composition and amount given in the example above. In addition, to be able to synthesize homogeneous and stable Alumina sol, the precursor AlP, the temperature and mixing time, as well as the added water and H₂SO₄ or HNOs or HCl, evaulated specifically to desired catalyst formulation. The most significant step in the synthesis of highly active and selective catalyst is the preparation of alumina sol (step ii) in which aluminum isopropoxide (AIP) and deionized water are mixed at room temperature and then, the temperature of the mixture is increased to 80-90°C in 15-30 min with 1000-1500 rpm of stirring rate and then, the mixture is kept at 80-90°C with the same stirring rate for 15-120 min. Then, the amount of sulfuric acid (or hydrochloric acid or nitric acid) given in the example above is added to obtain alumina sol. In the invention, the step of adding AIP in deionized water at room temperature makes sure that the formation of boehmite crystalline phase of alumina is prevented before incorporating silica sol; thus, enabling the control of homogeneous mixing of alumina sol and silica sol to form molecularly homogeneous mixed oxide with the crystallite sizes less than 20 nm after calcination (as seen in Table 1). In sol-gel methods of making alumina from AIP known in the literature, boehmite phase in alumina sol is intented to be formed; hence, AIP is always directly added in water at 80-100 °C before adding an acid (usually nitric acid used in known technique of the sol-gel in the literature to form a alumina sol). Unfortunately, generally known sol-gel method making the alumina sol to prepare alumina-silica mixed oxide supported nickel catalysts does not yield the intented final catalytic activity and selectivity for converting microalgae into biofuels/biochemical in seawater without dewatering and harvesting. In fact, in the present invention, the effect of the acid types, such as sulfiric acid, nitric acid and hydrochloric acid, on the acitivity and selectivity of the catalyst of the invention shows that sulfuric acid usage in the sol-gel method with the mentioned molar ratios in Example I yields stable alumina sol in addition to ultimately resulting in significantly active catalyst after calcination step in the conversion of microalgae and selectivity to biofuels and biochemicals.

In the invention method, step vi is a drying of the catalyst and even though it is used in all catalyst preparation methods, the reaction temperature and times have significant impact on crystal structures and/or crystallite sizes which influences subsequently applied calcination and/or reduction process steps. Step iv is therefore specific to the high loads of nickel (e.g. up to 98 wt.%) on silica/alumina (Ni-Al₂O₃-SiO₂) catalysts, which is the subject of the present invention. Besides, step (viii) is the most crucial step in obtaining highly active final catalysts. Without step (viii), the activity of the final catalyst is less than half of the activity of the catalyst treated with step (viii).

Using the method of the invention, a heterogeneous Ni-Al₂O₃-SiO₂ catalyst can directly convert the microalgae, *Nannochloropsis oculata,* in seawater into the biofuel and biochemicals without the requirement of high cost dewatering and harvesting processes. In one example of the application of the invention, the direct conversion of 6.0 wt% microalgae in seawater to biofuel and biochemicals was carried out in a batch reactor at 80 °C (it could be as low temperature as 50 °C or as high as 180 °C) and 1.0 atm (pressure could be 0.5-1.2 atm) for 24 h. 0.25 g of a catalyst was added to 25 ml of 6.0 wt% *N. oculata* in seawater and mixed at a constant stirring speed of 330 rpm to avoid mass transfer limitations. More than 93% of *N. oculata* in seawater was converted to a range of hydrocarbons and sugars and polyols.

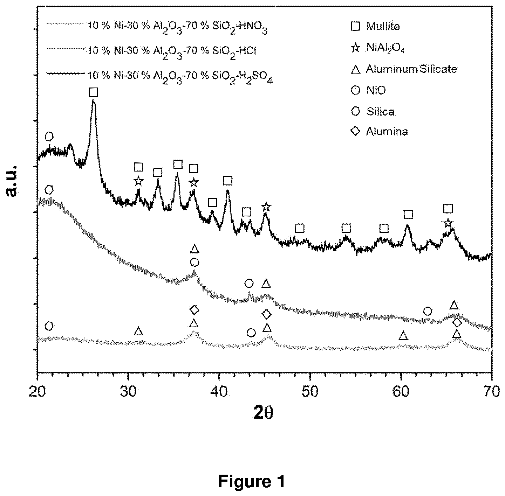

According to Figure 1, the crystallite size of nickel aluminate and mullite were calculated as around 11 nm and 16 nm in 10 % Ni- 30 % Al₂O₃- 70 % SiO₂-H₂SO₄ and 10 % Ni- 30 % Al₂O₃- 70 % SiO₂-SW, respectively. This shows that treatment of 10% Ni- 30 % Al₂O₃- 70 % SiO₂-H₂SO₄ catalyst with sodium chloride did not yield formation of new crystalline phases and change in sizes within the detection limits of wide-angle X-Ray Diffractometer. Moreover, the crystallite sizes of alumina-silicate phases in 10 % Ni- 30 % Al₂O₃- 70 % SiO₂-HCl and 10 % Ni- 30 % Al₂O₃- 70 % SiO₂-HNO₃ were calculated as 4 and 6 nm, respectively. Also, the size of nickel oxide crystallite was found as around 50 nm for 10 % Ni- 30 % Al₂O₃- 70 % SiO₂-HCl; while it was found as value less than 3 nm for 10 % Ni- 30 % Al₂O₃- 70 % SiO₂-HNO₃ because broadening of the diffraction peak of nickel oxide was too wide (at 2θ=43°) to be able to calculate the crystallite size.

The crystallite sizes in the catalysts, prepared using sulfuric acid, hydrochloric acid and nitric acid with the ratios given in the Example I, are given in Table 1;

**Table 1. Comparison of different catalyst's crystallite sizes**

| **Catalysts** | **Crystallite size of NiAl₂O₄** | **Crystallite size of Mullite** | **Crystallite size of NiO** | **Crystallite size of Al₂O₃** | **Crystallite size of Aluminum Silicate** |
|---|---|---|---|---|---|
| The catalyst prepared with sulfuric acid; 10 % Ni- 30 % Al₂O₃- 70 % SiO₂-H₂SO₄ | 11.2 | 15.9 | - | - | - |
| The catalyst prepared with sulfuric acid (after processed in seawater); 10 % Ni- 30 % Al₂O₃- 70 % SiO₂-SW | 10.9 | 15.7 | - | - | - |
| The catalyst prepared with HCl; 10 % Ni- 30 % Al₂O₃- 70 % SiO₂-HCl | - | - | 49.9 | - | 4 |
| The catalyst prepared with HNO₃; 10 % Ni- 30 % Al₂O₃- 70 % SiO₂-HNO3 | - | - | <3 | 6 | - |

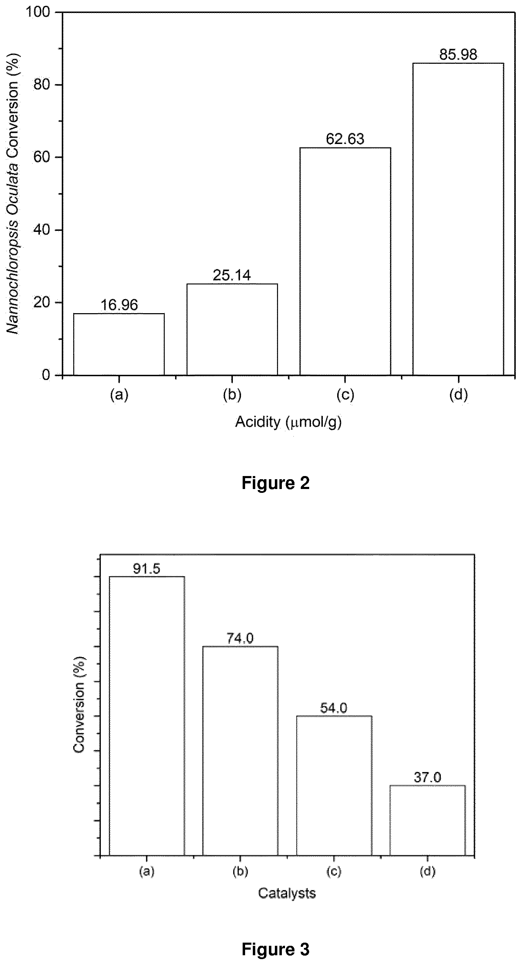

According to Figure 2, the highest microalgae conversions, higher than 70% were obtained at relatively low acidity, 15-25 µmol/g. In contrast to this behavior, lower microalgae conversions were observed at total acidity higher than 60 µmol/g. Indeed, the highest microalgae conversion was obtained over 10% Ni-30% Al₂O₃-70% SiO₂-SW catalyst which had total acidity of 25.14 µmol/g while the lowest microalgae conversion was observed over 10% Ni-30% Al₂O₃-70% SiO₂-HCl catalyst which had total acidity of 85.98 µmol/g; thus, it could be said that low total acidity was necessary to obtain high microalgae conversion. The desorption peak temperatures of 10% Ni-30% Al₂O₃-70% SiO₂-H₂SO₄ catalyst altered between 130-730 °C, whereas the acidic strengths at 600-730 °C were vanished after the treatment of the catalyst with NaCl and the acidic strength distribution shifted to range of 130-380 °C. That's why, it could be said that high microalgae conversion was observed over the catalysts which had low total acidity and acidic strength altering between the range of 130-380 °C. Also, to prove the exigency of nickel on catalyst, TPD-NH₃ analysis performed for 70% SiO₂-30% Al₂O₃-H₂SO₄ catalyst. It was founded that it had total acidity of 19.48 µmol/g and acidic strength at roughly 165 °C and it did not yield any microalgae conversion even if TPD results were very close to the results of 10% Ni-30% Al₂O₃-70% SiO₂-SW. This means that not only total acidity and acidic strength was effective but also nickel must be present to increase the catalytic activity.

According to Figure 3, the catalytic activities of prepared catalysts in the conversion of 6 wt. % *Nannchloropsis Oculata* in sea water at 80oC and 1 atm for 24 h. were illustrated in Figure 3. There were no N. Oculata conversion observed at same reaction conditions in the absence of catalyst and in the presence of 30% Al₂O₃-70 % SiO₂-H₂SO₄ catalyst which did not have any nickel in its structure. on 10% Ni- 30% Al₂O₃-70% SiO₂-HCl and 10% Ni- 30% Al₂O₃- 70% SiO₂-HNO₃ catalysts, microalgae conversion at 80oC under atmospheric pressure was obtained as 37% and 54%, respectively. The conversion of microalgae increased to 74% over 10% Ni- 30% Al₂O₃-70% SiO₂-H₂SO₄ catalyst. Surprisingly, the highest conversion, 91.5 % was obtained over 10% Ni- 30% Al₂O₃-70% SiO₂-H₂SO₄-SW catalyst.

### REFERENCES

**[1]** panelL.G.RobertsT.J.Patterson, A. links open overlay, L.G.Roberts, T.J.Patterson, & AbstractBiofuels are renewable transportation fuels produced from biomass. Biofuels must be compatible with existing engine and fuel requirements; there are often similar properties between biofuels and their conventional petroleum counterparts. Ethanol i. (2014, April 14). Biofuels. Encyclopedia of Toxicology (Third Edition). Retrieved December 20, 2022, from https://www.sciencedirect.com/science/article/pii/B978012386454301054X
**[2]** Fossil fuel air pollution responsible for 1 in 5 deaths worldwide. C-CHANGE | Harvard T.H. Chan School of Public Health. (2021, February 9). Retrieved December 20, 2022, from https://www.hsph.harvard.edu/c-change/news/fossil-fuel-air-pollution-responsible-for-1-in-5-deaths-worldwide/
**[3]** Author links open overlay panelAna I.BarrosAna L.GonçalvesManuelSimõesJosé C.M.PiresPersonEnvelope, I.Barros, A., L.Gongalves, A., ManuelSimdes, C.M.PiresPersonEnvelope, J., & Abstract Research studies on microalgae have increased in the last decades due to the wide range of applications associated to these photosynthetic microorganisms. Microalgae are an important source of oils and other biomolecules that can be used in the pr. (2014, October 21). Harvesting techniques applied to microalgae: A Review. Renewable and Sustainable Energy Reviews. Retrieved December 20, 2022, from https://www.sciencedirect.com/science/article/abs/pii/S1364032114008107
**[4]** Dewatering of microalgal cultures: A major bottleneck to algae-based fuels. (n.d.). Retrieved December 20, 2022, from https://www.researchgate.net/publication/245308669_Dewatering_of_microalgal _cultures_A_major_bottleneck_to_algae-based_fuels
*[5]* Application of the sol-gel methods to catalyst preparation - researchgate. (n.d.). Retrieved January 3, 2023, from https://www.researchgate.net/publication/222590797_Application_of_the_Sol-Gel Methods to Catalyst_Preparation
[6] Zuorro, A., Garcia-Martinez, J. B., & Barajas-Solano, A. F. (2020, December 28). The application of catalytic processes on the production of algae-based biofuels: A Review. MDPI. Retrieved December 21, 2022, from https://www.mdpi.com/2073-4344/11/1/22

## Claims

1. A synthesis method of heterogeneous catalyst formulation with high catalytic activity and selectivity to be used in direct conversion of microalgae into biofuels without harvesting and dewatering and by one-step sol-gel method comprises the following steps,
i. synthesizing silica sol by mixing 1-5 M hydrochloric acid (HCl, 36.5-38 wt.%) with the precursor of tetraethylorthosilicate (≥ 98% purity), ethanol (≥99.8 % purity), and deionized water at 60-90 °C for 1-4 hours
ii. synthesizing alumina sol by mixing precursor of aluminum isopropoxide (AIP, ≥ 98% purity), deionized water at room temperature,
iii. increasing the temperature to 80-90°C in 15-30 min with 1000-1500 rpm of stirring rate and adding sulfuric acid (H₂SO₄, ≥98 purity) or hydrochloric acid or nitric acid (68 wt.%) to obtain the alumina sol,
iv. mixing silica and alumina sols, and adding nickel (II) acetate hydrate (>99% purity) to the silica-alumina sol mixture,
v. evoporating the excess solvent at 50-80°C under vacuum or inert flow for 30-120 min, and obtaining a gel,
vi. drying the gel at 100-150°C for 3-24 h, and calcinating the gel at 500-900°C for 6-24 h with 10°C/min heating rate, obtaining a catalyst which has formulation of Ni-Al₂O₃-SiO₂
vii. after calcination, ball milling or hand grinding and sieving of the catalyst to mesh size less than 200 for catalytic reactions
viii. sieved catalysts are pretreated with sodium chloride (1-5 wt.%) containing water at 70-90 °C for 12-48 h, and drying at 100-150 °C for 3-24 h

2. The method according to Claim 1, **characterized by** comprising following steps:
i. synthesizing silica sol by mixing 1 M hydrochloric acid (HCl, 38 wt.%) with the precursor of tetraethylorthosilicate (≥ 98% purity), ethanol (≥99.8 % purity), and deionized water at 80 °C for 2 hours
ii. synthesizing alumina sol by mixing precursor of aluminum isopropoxide (AlP, ≥ 98% purity), deionized water at room temperature,
iii. increasing the temperature to 85°C in 30 min with 1500 rpm of stirring rate and then adding sulfuric acid (H₂SO₄, ≥98 purity) or hydrochloric acid or nitric acid (68 wt.%),
iv. mixing silica and alumina sols, and adding nickel (II) acetate hydrate (>99% purity) to the sol mixture,
v. evoporating the excess solvent at 70°C, and obtaining a gel,
vi. drying the gel at 120°C for 12 h, and calcinating the gel at 900°C for 6 h with 10°C/min heating rate, obtaining a catalyst which has formulation of Ni-Al₂O₃-SiO₂,
vii. after calcination, grinding and sieving of the catalyst to mesh size of less than 200 mesh for catalytic reactions,
viii. sieved catalysts are pretreated with sodium chloride (3.5 wt.%) containing water at 90 °C for 24 h, and drying at 120 °C for 12 h,

3. The method according to Claim 1 or 2, wherein the microalgae is *Nannochloropsis oculata, Chlorella minutissima, Chlorella vulgaris, Botryococcus braunii,* or *Spirulina.*

4. The method according to Claim 1 or 2, wherein the proportion of water to aluminum (H₂O/Al) in step (ii) is 115.5 mole, the proportion of sulfuric acid to aluminum (H₂SO₄/Al) in step (ii) is 2.42 mole.

5. The method according to Claim 1 or 2, wherein the proportion of ethanol to silica (EtO/Si) in step (i) is 3.81 mole, the proportion of water to silica (H₂O/Si) in step (i) is 4.69, and the proportion of hydrochloric acid to silica (HCl/Si) in step (i) is 7.30 × 10⁻⁴.

6. A heterogeneous catalyst synthesized by the method according to Claim 1 or 2.

7. A heterogeneous catalyst for direct conversion of microalgae into biofuels, **characterized by** comprising 5-95% Al₂O₃, 95-5% SiO₂ and 0.5-30% Ni by weight.

8. A heterogeneous catalyst for direct conversion of microalgae into biofuels, **characterized by** comprising 70% Al₂O₃, 30% SiO₂ and 10% Ni by weight.

9. A heterogeneous catalyst according to Claim 7 or 8, wherein the microalgae is *Nannochloropsis oculata, Chlorella minutissima, Chlorella vulgaris, Botryococcus braunii,* or *Spirulina.*

10. Use of a heterogeneous catalyst according to Claim 9 in conversion of microalgae into biofuel without harvesting and dewatering.
